(19) Europäisches Patentamt
European Patent Office
Office européen des brevets

(11) **EP 2 682 402 A1**

(12) **EUROPEAN PATENT APPLICATION**
published in accordance with Art. 153(4) EPC

(43) Date of publication:
**08.01.2014 Bulletin 2014/02**

(51) Int Cl.:
*C07K 14/415* (2006.01)     *A23L 1/305* (2006.01)
*A61K 8/64* (2006.01)     *A61K 8/97* (2006.01)
*A61Q 19/00* (2006.01)     *C07K 16/16* (2006.01)
*C12N 1/04* (2006.01)

(21) Application number: **12754617.4**

(22) Date of filing: **02.03.2012**

(86) International application number:
**PCT/JP2012/055460**

(87) International publication number:
**WO 2012/121172 (13.09.2012 Gazette 2012/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.03.2011 JP 2011048318**

(71) Applicant: **Kaneka Corporation
Osaka 530-8288 (JP)**

(72) Inventors:
• **KAWAHARA, Hidehisa
Suita-shi
Osaka 564-8680 (JP)**

• **KEGASA, Hideaki
Takasago-shi
Hyogo 676-8688 (JP)**
• **ARAI, Naoki
Takasago-shi
Hyogo 676-8688 (JP)**

(74) Representative: **HOFFMANN EITLE
Patent- und Rechtsanwälte
Arabellastrasse 4
81925 München (DE)**

(54) **ICE CRYSTALLIZATION INHIBITOR DERIVED FROM PLANT SEED**

(57)     The objective to be solved by the present invention is to provide a novel ice crystallization inhibitor which is industrially useful, which can be efficiently and stably produced in a safe process suitable for a food production without difficulty and which has excellent functions and properties. Also, the objective of the present invention is to provide an antibody which specifically reacts with the ice crystallization inhibitor, and a composition, a food, a biological sample protectant and a cosmetic which contain the ice crystallization inhibitor. Furthermore, the objective of the present invention is to provide a peptide which gives an indication of a protein having an ice crystallization inhibitory activity. The ice crystallization inhibitor according to the present invention is characterized in comprising a seed protein derived from a plant belonging to genus Vigna in Leguminosae, an allied species thereof or an improved species thereof. The ice crystallization inhibitor according to the present invention is characterized in comprising a seed protein derived from a plant belonging to genus Vigna in Leguminosae, an allied species thereof or an improved species thereof.

EP 2 682 402 A1

**Description**

TECHNICAL FIELD

The present invention relates to an ice crystallization inhibitor which is derived from a plant seed; an antibody which specifically responds with the ice crystallization inhibitor; a composition, a food, a biological sample protectant and a cosmetic which contain the ice crystallization inhibitor; and a peptide which provides an indication of a protein having an ice crystallization inhibitory activity.

BACKGROUND ART

It is known that an organism which lives under the condition of low temperature produces an ice crystallization inhibitor such as an antifreeze protein and uses such an ice crystallization inhibitor to protect itself from freezing of the cells. Hereinafter, an antifreeze protein is referred to as "AFP". AFP is a protein which has functions such as thermal hysteresis, and can inhibit an aqueous solution from freezing and control the figure of ice crystal. AFP has been found from an organism such as a fish, an insect, a plant, a fungi and a microorganism.
In the past, AFP has been found in a fish such as a Cottidae, an insect such as a mealworm, a microorganism such as Flavobacterium and the like, and the AFPs have a high ice crystallization inhibitory activity (Patent Documents 1 to 3). In addition, AFP also has been found in a plant such as a winter rye and a carrot (Non-patent Documents 1 and 2).
Furthermore, as AFP derived from a fungus, AFPs derived from a basidiomycete such as Typhula ishikariensis and Flammulina velutipes KUAF-1 are known (Patent Documents 4 and 5).
Recently, there was an attempt at using AFP for maintaining the quality of a frozen confection product and a frozen food, such as an ice cream, by industrially utilizing the above-described properties (Patent Documents 6 and 7).

PRIOR ART DOCUMENT

PATENT DOCUMENT

| Patent Document 1: | JP 2004-83546 A |
| Patent Document 2: | JP 2002-507889 T |
| Patent Document 3: | JP 2004-161761 A |
| Patent Document 4: | JP 2004-24237 A |
| Patent Document 5: | JP 2004-275008 A |
| Patent Document 6: | WO 92/22581 |
| Patent Document 7: | WO 94/03617 |

NON-PATENT DOCUMENT

Non-Patent Document 1: Plant Physiology, vol. 119, pp. 1361-1369 (1999)
Non-Patent Document 2: Biochem. J., vol. 340, pp. 385-391 (1999)

SUMMARY OF THE INVENTION

PROBLEMS TO BE SOLVED BY THE INVENTION

Regardless of the above-described properties, it is difficult to completely remove an odor from AFP derived from a fish. In addition, AFP derived from an insect and a microorganism is not suitable for a food, since it is difficult to use an insect and a microorganism as a raw material for a food.
On the other hand, AFP derived from a plant is expected to be used for a food, since a plant is used as a raw material of a food. However, AFP derived from a plant has been rarely used industrially, since the binding ability thereof to an ice is weaker than those of other AFPs and the APF is not sufficiently stable against heat.
Under the above-described situation, the objective to be solved by the present invention is to provide a novel ice crystallization inhibitor which is industrially useful, which can be efficiently and stably produced in a safe process suitable for a food production without difficulty and which has excellent functions and properties. Also, the objective of the present invention is to provide an antibody which specifically reacts with the ice crystallization inhibitor, and a composition, a food, a biological sample protectant and a cosmetic which contain the ice crystallization inhibitor. Furthermore, the objective of the present invention is to provide a peptide which gives an indication of a protein having an ice crystallization inhibitory activity.

SOLUTIONS TO THE PROBLEMS

The present inventors intensively studied so as to solve the above problems. As a result, the inventors completed the present invention by finding that an ice crystallization inhibitor which has excellent properties can be obtained from a seed of a plant belonging to genus Vigna in Leguminosae.

The ice crystallization inhibitor of the present invention is characterized in comprising a seed protein derived from a plant belonging to genus Vigna in Leguminosae, an allied species thereof or an improved species thereof.

As the plant belonging to genus Vigna in Leguminosae, an adzuki bean or a mung bean is preferred. The present inventors experimentally found that a seed of the above plants may contain a seed protein which has very excellent ice crystallization inhibitory activity.

As the seed protein, a grain filling protein, a LEA protein: Late embryogenesis abundant protein, and a protein having an amino-acid sequence of SEQ ID NO: 1 or SEQ ID NO: 2, or an amino-acid sequence corresponding to the sequence of SEQ ID NO: 1 or SEQ ID NO: 2 with one or more amino-acid deletions, substitutions or additions and containing an indicative sequence of a protein having an ice crystallization inhibitory activity are preferred. The excellent ice crystallization inhibitory activity of the above seed proteins is experimentally demonstrated by the present inventors.

The seed protein which is contained in the ice crystallization inhibitor according to the present invention may form a complex with other protein. In the seed protein which was found by the present inventors and which exhibits an ice crystallization inhibitory activity, there is a protein which has a structural feature of a complex with other denatured protein.

The antibody according to the present invention is characterized in specifically responding with the above-described ice crystallization inhibitor.

The composition, food, biological sample protectant and cosmetic according to the present invention are characterized in comprising the above-described ice crystallization inhibitor of the present invention.

The peptide according to the present invention is characterized in having a sequence of SEQ ID NO: 2, or an amino-acid sequence corresponding to the sequence of SEQ ID NO: 2 with one or more amino-acid deletions, substitutions or additions and containing an indicative sequence of a protein having an ice crystallization inhibitory activity.

EFFECT OF THE INVENTION

The ice crystallization inhibitor according to the present invention can be stably supplied, since the ice crystallization inhibitor can be efficiently produced from a seed of a plant belonging to genus Vigna in Leguminosae, an allied species thereof or an improved species thereof with no difficulty. In addition, the ice crystallization inhibitor can be used for a food at ease, since the ice crystallization inhibitor has no problem of odor and is obtained from a plant. Therefore, for example, the ice crystallization inhibitor according to the present invention can be used for maintaining a quality of a frozen food or the like. In addition, the ice crystallization inhibitor can be effectively used as a cosmetic, a protectant when an organ, a cell, blood or platelet is preserved in a frozen state, or the like.

BRIEF DESCRIPTION OF THE DRAWING

Figure 1 is a magnified photograph of an ice crystal of which figure was controlled by the ice crystallization inhibitor according to the present invention.
Figure 2 is a magnified photograph of an ice crystal of which figure was controlled by the ice crystallization inhibitor according to the present invention.

MODES FOR CARRYING OUT THE INVENTION

Hereinafter, the present invention is described in more detail.

In the present invention, the term "ice crystallization inhibitor" means a substrate which adsorbs on a crystal face of an ice crystal and inhibits a growth of the ice crystal. In addition, as the result of the adsorption on a crystal face, free water is inhibited from further adsorbing to the ice crystal and ice recrystallization is prohibited. In other words, the ice crystallization inhibitor according to the present invention means a substance having an ice crystallization inhibitory activity which is confirmed by any one of public methods such as measurement of ice recrystallization inhibitory activity, measurement of a thermal hysteresis and observation of the structure of ice crystal.

For example, an ice crystallization inhibitory activity can be measured by the following condition. First, an aqueous solution of an ice crystallization inhibitor containing 30 w/v% sucrose is cooled down to -40°C, and then the temperature is raised up to -6°C. After 30 minutes, an average area of observed ice crystals is measured. As a control, 30 w/v% sucrose aqueous solution is similarly treated and an average area of ice crystals is measured. For example, an average area of ice crystals can be measured by analyzing a microscopic image using a commercially available image analysis process soft, such as Image Factory manufactured by Imsoft, ADOBE PHOTOSHOP ELEMENT 8 manufactured by Adobe and Scion Image manufactured by Scion, and dividing a total area of the ice crystals in the image by the number of the ice crystals in the image. Next, an ice crystallization inhibitory activity can be quantitatively evaluated by using a value obtained by dividing the measured average area of the ice crystals by the average area of the control as the indicator. Since an average area of ice crystals is smaller as an ice crystallization inhibitory activity is stronger, when the formulation of ice crystal is inhibited if only a little by adding the ice crystallization inhibitor in comparison with the control, the above-described value is smaller than 1. In such a case, it is judged that the ice crystallization inhibitor has an ice crystallization inhibitory activity.

A thermal hysteresis is expressed as a difference between a melting temperature of ice and a freezing temperature of a solution in the presence of the ice crystallization inhibitor. The higher the value is, the higher the activity of the ice crystallization inhibitor is. For example, a thermal hysteresis is measured by the following condition.

A glass petri dish is maintained in a temperature of -20°C using a phase contrast microscope which can control a temperature in a low temperature range, 1 mL of a sample is placed on the glass petri dish, and a temperature is decreased to -40°C at a speed of 100°C/min to form ice crystal. The ice crystal is warmed up to -5°C at a speed of 100°C/min, and then further warmed up at a speed of 5°C/min to be a single crystal. Next, the temperature is decreased at a speed of 1°C/min, and the time at which the single crystal starts to grow is measured. A thermal hysteresis can be calculated in accordance with the following formula.

```
Thermal hysteresis (°C) = [60 - 1] (°C/sec) × measured time (sec)
```

When the calculated value is 0.02°C or more, it is judged that a measured sample has an ice crystallization inhibitory activity. A thermal hysteresis is preferably not less than 0.025°C and more preferably not less than 0.03°C.

Also, it can be judged by observing a structure of ice crystal in water or a solution which contains a test sample whether the test sample is an ice crystallization inhibitor or not. Specifically, a figure of ice crystal which is obtained by cooling usual water is like a flat disc. On the other hand, an ice crystal which is obtained by adding an ice crystallization inhibitor takes various forms such as hexagonal-shaped form, i.e. flat hexagonal column form, and bipyramid form depending on a crystal face of an ice crystal on which the inhibitor adsorbs. Therefore, when a form of an ice crystal is other than flat disc form due to an addition of a substance, it is judged that the substance is an ice crystallization inhibitor.

The ice crystallization inhibitor according to the present invention contains a seed protein derived from a plant belonging to genus Vigna in Leguminosae, an allied species thereof or an improved species thereof.

A seed of a plant belonging to genus Vigna in Leguminosae is exemplified by, in the Japanese name, a large-size adzuki bean (Dainagon adzuki bean) such as Hokuto Dainagon, Toyomi Dainagon, Akane Dinagon, Kamui Dinagon, Beni Dainagon and Sahoroshouzu; a normal-size adzuki bean such as Erimoshouzu, Shumarishouzu, Kitanootome and Sahoroshouzu; a white adzuki bean such as Kitahotaru and Hokkaishiroshouzu; a mung bean; a black-eyed pea. An adzuki bean is preferred and a large-size adzuki bean is more preferred among the above examples, since the beans are widely eaten for a long time and readily available. In addition, an ice crystallization inhibitory activity and a thermal hysteresis activity per unit weight of an extract obtained from the beans are excellent.

For example, the term "allied species of a plant belonging to genus Vigna in Leguminosae" in the present invention means a breed of a plant which similarly belongs to family Leguminosae but does not belong to genus Vigna and which is close to genus Vigna in an academical classification. A specific allied species of a plant belonging to genus Vigna in Leguminosae means a breed of a plant which is close to genus Vigna in Leguminosae in an academical classification. The term "improved species of a plant belonging to genus Vigna in Leguminosae" means a specific plant belonging to genus Vigna in Leguminosae improved by artificial selection, hybridization, mutation, gene recombination and the like.

The above seed protein is not particularly limited as long as the protein is a seed protein extracted from the above plant, and may be a fraction which contains the seed protein. Specifically, the above seed protein may be various grain filling proteins. Such a grain filling protein is generally defined as a protein which is synthesized and accumulated during a process of grain filling. The term "grain filling" means that a plant seed matures and enlarges. Such a grain filling protein is exemplified by various seed storage proteins such as albumin, globulin, glutelin and prolamin; a stress protein such as a heat shock protein; and a LEA protein (Late embryogenesis abundant protein). In particular, a LEA protein is preferred in the above grain filling proteins.

The ice crystallization inhibitor according to the present invention may contain only one of the above seed proteins or a plurality of the above seed proteins. The above seed protein may form a complex with other protein in the ice crystallization inhibitor of the present invention. It is particularly known that a LEA protein interacts with other denatured protein. A denatured protein which forms a complex with the seed protein according to the present invention may be derived from a seed containing the seed protein or derived from other than the seed. In addition, the number of the denatured protein may be only one or not less than two.

The above LEA protein is a group of highly hydrophilic proteins of which expression is specifically induced when a water stress is produced in a cell, and is synthesized and accumulated during a process of grain filling, which means maturing and enlargement of a plant seed. It is sometimes observed that a LEA protein is accumulated in a leaf, a root, pollen or the like other than a seed. A protein which is contained in other than a seed is included in the range of the present invention as long as the protein has a homology with a seed protein of a plant belonging to genus Vigna in Leguminosae and is an ice crystallization inhibitor.

A molecular weight of the ice crystallization inhibitor according to the present invention is not particularly limited, and for example, an average molecular weight measured by gel filtration chromatography is preferably not less than 1 kDa and not more than 200 kDa. When the molecular weight is 1 kDa or more, an ice crystallization inhibitory activity may be sufficiently exhibited. On the other hand, when the molecular weight is too large, a viscosity of the solution may become too high in some cases. Therefore, the molecular weight is preferably 200 kDa or less. The molecular weight is more preferably not more than 180 kDa and even more preferably not more than 160 kDa. The ice crystallization inhibitor may be possibly a complex, and the above average molecular weight may be an average molecular weight of a monomer of the complex. In addition, various decomposed substances of the ice crystallization inhibitor which has the above molecular weight are also included in the range of the present invention as long as such decomposed substances exhibit an ice crystallization inhibitory activity and the like.

It is preferred that an amino-acid sequence of the ice crystallization inhibitor of the present invention is each of sequences of SEQ ID NO: 1 to SEQ ID NO: 3, or an amino-acid sequence corresponding to each of sequences of SEQ ID NO: 1 to SEQ ID NO: 3 with one or more amino-acid deletions, substitutions or additions and containing an indicative sequence of a protein having an ice crystallization inhibitory activity.

In the above ice crystallization inhibitor, the number of an amino-acid which is deleted, substituted or added is preferably not less than 1 and not more than 5, more preferably not less than 1 and not more than 3, and even more preferably 1 or 2.

In the above ice crystallization inhibitor, the sentence of "containing an indicative sequence of a protein having an ice crystallization inhibitory activity" means that the protein having a sequence with deletion, substitution or addition has an ice crystallization inhibitory activity which is measured by any one of publicly known method such as a measurement of an ice recrystallization inhibitory activity , a measurement of a thermal hysteresis and an observation of an ice crystal structure.

As a method for obtaining the ice crystallization inhibitor of the present invention, a method of extracting from a seed of the above-described plant belonging to genus Vigna in Leguminosae is exemplified. Hereinafter, a specific production method is described.

The ice crystallization inhibitor may be further induced by preliminarily acclimatizing a seed of a plant belonging to genus Vigna in Leguminosae which is subjected to extraction to low temperature. For example, a seed may be stored at 20°C or lower for 3 days or more. However, the ice crystallization inhibitor can be sufficiently obtained from a seed of a plant belonging to genus Vigna in Leguminosae even without acclimatizing to low temperature.

The form of a seed of a plant belonging to genus Vigna in Leguminosae which is subjected to extraction may be a fractured form, a crushed form, a grinded form or the like. The fractured form or the like may be obtained by fracturing a raw seed or a dried seed, or the like.

An extraction efficiency can be enhanced by fracturing a seed or the like. As a means for fracturing a plant seed, various general means can be applied. For example, a physical fracturing means such as pressurized fracture, mechanical grinding, sonication and homogenizer can be used. A specific fracturing means is exemplified by a homogenizer such as Potter-Elehjem homogenizer, a blender such as Waring blender, a mil such as dyno mil, French press, a mortar and a muddler, a pulverizing machine, freezing and pulverization by using liquid nitrogen, and sonication treatment.

After a seed is roughly fractured, the fractured seed may be further grinded or finely crushed using a mortar and a muddler, a ball mil, a hammer mil or the like.

A component is extracted by dispersing a fractured seed or the like in an extracting solvent. When a dried seed is used, it is preferred that a dried seed is preliminarily immersed in an extracting solvent in order to readily extract a component.

It is preferred that the target substance is hardly denatured due to an extracting solvent and toxicity of an extracting solvent is low. A preferable extracting solvent is exemplified by water; brine; a buffer solution such as a sodium acetate buffer solution, a phosphate buffer solution and a tris-hydrochloric acid buffer solution; a hydrophilic organic solvent such as methanol, ethanol and acetone; other organic solvent such as ethyl acetate; and a mixed solvent of the above solvents. When it is taken into account that the ice crystallization inhibitor of the present invention is applied to a food, a preferred solvent is water, brine, a sodium acetate aqueous solution or an arbitrarily-mixed solution of the solvents. A composition, pH or the like of an extracting solvent may be arbitrarily selected. In general, extracting solvent is preferably adjusted to be neutral, for example, the pH is adjusted to about not less than 5 and not more than 9.

An amount of an extracting solvent relative to a seed may be arbitrarily selected, and is generally adjusted so that a seed is totally immersed in the extracting solvent. For example, an amount of an extracting solvent relative to a used seed may be approximately adjusted to not less than 1 mL/g and not more than 5 mL/g.

For extraction, a raw seed or a dried seed may be mixed with an extracting solvent, and the seed may be fractured in the extracting solvent using a mixer or the like. In addition, a seed may be immersed only, extraction may be carried out in a reduced pressure, or a mixture may be stirred during extraction.

A temperature for extraction may be arbitrarily adjusted. For example, a temperature for extraction may be not less than 0°C and not more than 100°C, and more preferably not less than 2°C and not more than 10°C. In particular, when the temperature is 10°C or lower, degradation of a protein may be inhibited more surely by decreasing an activity of a protease which is contained in a seed. With respect to regulation of temperature, a temperature of a mixture of a seed and an extracting solvent may be adjusted to a predetermined temperature for extraction, or an extracting solvent of which temperature is adjusted to a predetermined temperature may be added and an extraction may be carried out with maintaining the temperature. For example, after a seed is immersed in water or the like and the mixture is maintained at a temperature of not less than 2°C and not more than 10°C for a sufficient time, water is substituted by an extracting solvent and extraction carried out with fracturing the seed.

A time for extraction may be also arbitrarily adjusted, and is generally not less than 5 minutes and not more than 100 hours.

After extraction, obtained extract may be separated by filtration, centrifugation or the like, and the separated extract may be used as the ice crystallization inhibitor. Further, an extraction residue is repeatedly subjected to a similar extraction treatment, the obtained extracts are brought together, and the extract may be used as the ice crystallization inhibitor.

An alkaline treatment step in which a base is added to the obtained extract may be carried out. By such an alkaline treatment step, an ice crystallization inhibitory activity of a protein may be increased in some cases.

A condition of an alkaline treatment is not particularly limited and may be arbitrarily set. For example, as a base, an alkali metal hydroxide such as sodium hydroxide and potassium hydroxide may be used. The value of pH is preferably not less than 10.0 and more preferably not less than 10.5. The upper limit of pH is not particularly limited, and pH is preferably not more than 13.0 and more preferably not more than 12.0.

After the pH is adjusted to higher as the above, the mixture may be neutralized using hydrochloric acid or the like so that the pH may be adjusted not less than about 6.0 and not more than about 8.0.

The ice crystallization inhibitor obtained as the above may be further purified as necessary. For example, a solid substance may be removed by a preferable combination of decantation, filtration, centrifugation and the like. In addition, for example, precipitation by salting-out or using an organic solvent, affinity chromatography, ion-exchange column chromatography, gel filtration, purification by binding to ice using a low-speed refrigerator, concentration by dialysis or ultrafiltration, and the like may be properly combined.

A form of the ice crystallization inhibitor of the present invention may be variously selected depending on the use application, and may be a solid, a solution, a concentrated liquid, a dispersion or the like. As necessary, the ice crystallization inhibitor may be further formed into an arbitrary form such as a powder, a granule or a tablet. A method of formulation is not particularly limited, and is exemplified by a method for powderizing the above extract with a conventional method such as spray drying and freeze drying, a method for powderizing or granulating the extract by adsorbing and supporting the extract on an excipient, and the like. Such methods are well-known for the person skilled in the art, and the skilled person can select a proper method to be used depending on the intended use application.

The ice crystallization inhibitor according to the present invention can be utilized for the purpose of removing impediment caused by crystallization of water in various fields where such an impediment is present. For example, the inhibitor can be utilized in the fields of a food, machinery, civil engineering, cosmetics, and medicine in which a biological sample is used.

The ice crystallization inhibitor of the present invention may have various forms depending on the use application thereof. The ice crystallization inhibitor may be used as it is, or may be in the form of a solution, a concentrated solution, a suspension, a freeze-dried product, a powder, a granule, a tablet and the like. In addition, the ice crystallization inhibitor may be mixed with an excipient to be used as a composition.

The antibody according to the present invention reacts specifically with the above-described ice crystallization inhibitor. The antibody therefore can be used for confirming the presence or absence of the ice crystallization inhibitor in a basidiomycete or the culture medium therefor, and specifying a polysaccharide having an ice crystallization inhibitory activity from a culture medium for a basidiomycete.

The antibody according to the present invention may be produced according to a conventional method. For example, a mouse, rat or the like is immunized with the above-described ice crystallization inhibitor, and the antibody-producing cell or the splenocyte is fused with a myeloma cell to obtain a hybridoma. The hybridoma is cloned, and the clone producing an antibody which is reactive specifically with the above-described ice crystallization inhibitor is screened. The clone is cultured, and a secreted monoclonal antibody may be purified.

In the field of a food, it is possible to prevent the deterioration of taste and others by suppressing ice crystallization of water contained in a food. For example, when water in a food is crystallized to be an ice, protein, fat component, oil component and the like are physically compressed, and the structure of the components is changed. As a result, taste, quality and the like of a food is deteriorated. When the ice crystallization inhibitor is added to a food, such deterioration can be inhibited and the quality of a frozen food and the like can be improved.

In the fields of machinery and civil engineering, the ice crystallization inhibitor according to the present invention can be utilized as a cryoprotective agent for movable part of machinery, road, ground and the like.

In the field of cosmetics, the ice crystallization inhibitor according to the present invention can be utilized as an additive agent for preventing the reduction in quality of cosmetics. For example, when cosmetics containing an oil component or a fat component are frozen, water contained in the cosmetics is crystallized to be an ice. As a result, the oil component and fat component may be physically compressed and the structure thereof may be destroyed, whereby the quality and impression from use of the cosmetics become deteriorated. When the ice crystallization inhibitor according to the present invention is used, the reduction in quality and the like can be avoided since ice crystallization of water is prevented and the structure of an oil component and a fat component is maintained.

In the field of medicine, the ice crystallization inhibitor according to the present invention can be utilized as a protectant when a biological sample is cryopreserved. For example, when a biological sample such as a cell, blood, an organ, a bioactive protein, a bioactive peptide and a low-molecular compound derived from a living body is cryopreserved in a conventionally and publicly known preservation solution, water in the preservation solution freezes to generate ice crystals. The ice crystals may damage the biological sample. On the other hand, when the ice crystallization inhibitor according to the present invention is added thereto, the biological sample can be protected from the damage caused by ice crystals since generation and growth of ice crystal can be suppressed.

The present application claims the benefit of the priority date of Japanese patent application No. 2011-048318 filed on March 4, 2011, and all of the contents of the Japanese patent application No. 2011-048318 filed on March 4, 2011, are incorporated by reference.

EXAMPLES

Hereinafter, the present invention is described in more detail with Examples. However, the present invention is not limited to the following Examples in any way.

In the following Examples, a concentration of a protein was measured by a bicinchoninic acid (BCA) method using a commercially available measurement kit (BCA Protein Assay, manufactured by Thermo SCIENTIFIC K. K.). A measurement condition was set in accordance with an ordinary method, and bovine serum albumin (BSA) was used as a standard protein.

Example 1

Into a 1, 000 mL beaker, commercially available Dainagon adzuki bean (100 g) was added. Distilled water was added until the bean was immersed and the mixture was maintained at 4°C for 48 hours. The distilled water was removed, and then 5 mM Tris-HCl buffer solution (pH: 8.0, 500 mL) was added thereto. The bean was fractured at 4°C using a mixer, and then the mixture was filtered using a gauze and centrifuged at 4°C and at 8,000 × g for 30 minutes. A supernatant (460 mL) after centrifugation was obtained as a crude extract. A concentration of protein in the obtained crude extract was measured by BCA method; as a result, the concentration was 42 mg/mL.

Example 2

To the crude extract (460 mL) obtained in Example 1, acetone which was cooled down to -30°C was added dropwise little by little until a concentration of acetone became 25 v/v%. Then, an acetone solution was separated from an insoluble component (insoluble fraction 1). An ice crystallization inhibitory activity of the insoluble fraction 1 was not detected. Next, acetone was added to the above acetone solution until an acetone concentration became 75 v/v%, and an insoluble component (insoluble fraction 2) was separated from 75 v/v% acetone solution. The obtained insoluble fraction 2 was dissolved in 5 mM Tris-HCl buffer solution (pH: 8.0, 100 mL). The obtained solution was subjected to dialysis using a dialysis membrane (molecular weight cut off: 14,000), and then the solvent was replaced with the same buffer solution to obtain a solution (230 mL) as an acetone fraction. A concentration of protein in the acetone fraction was measured by BCA method; as a result, the concentration was 8.3 mg/mL.

Example 3

The solution (230 mL) obtained in Example 2 was charged in a DEAE column (product name: DEAE TOYOPEARL 650M, manufactured by TOSOH Corporation) which was equilibrated using the above buffer solution, and eluted at a flow rate of 2.0 mL/min with NaCl gradient of 0 to 0.5 M to obtain non-adsorbed fraction as an active fraction. The obtained fraction was subjected to dialysis using a dialysis membrane (molecular weight cut off: 14,000), and then the solvent was replaced with 20 mM sodium acetate buffer solution (pH: 5.0) to obtain a solution (485 mL). A concentration of protein in the obtained DEAE column chromatography active fraction was measured by BCA method; as a result, the concentration was 1.8 mg/mL.

Example 4

The solution (485 mL) obtained in Example 3 was charged in a cation-exchange column (product name: CM TOYOPEARL 650M, manufactured by TOSOH Corporation) which was equilibrated using the above buffer solution, and eluted at a flow rate of 2.0 mL/min with NaCl gradient of 0 to 0.5 M to obtain an adsorbed fraction  as an active fraction. The obtained fraction was subjected to dialysis and desalination using a dialysis membrane (molecular weight cut off: 14,000), and then the solvent was replaced with 20 mM phosphate buffer solution (pH: 6.0) to obtain a solution (132 mL). A concentration of protein in the obtained active fraction (CM1 fraction) was measured by BCA method; as a result, the concentration was 0.73 mg/mL.

Example 5

The solution (132 mL) obtained in Example 4 was charged in a cation-exchange column (product name: CM TOYOPEARL 650M, manufactured by TOSOH Corporation) which was equilibrated using the above buffer solution, and eluted at a flow rate of 2.0 mL/min with NaCl gradient of 0 to 0.5 M to obtain a non-adsorbed column fraction (CM2 non-adsorbed fraction) and an adsorbed column fraction (CM2 adsorbed fraction) respectively. The adsorbed column fraction was subjected to dialysis and desalination using a dialysis membrane (molecular weight cut off: 14,000), and then the solvent was replaced with 20 mM phosphate buffer solution (pH: 6.0). Concentrations of protein in the obtained CM2 non-adsorbed fraction (126 mL) and CM2 adsorbed fraction (64 mL) were measured by BCA method; as a result, the concentrations were respectively 0.22 mg/mL and 0.29 mg/mL.

Example 6

The CM2 non-adsorbed fraction and CM2 adsorbed fraction obtained in Example 5 were respectively dissolved in 20 M phosphate buffer solution. Concentrations of protein of each obtained aqueous solution (1000 $\mu$L) were respectively 5000 $\mu$g/mL and 5000 $\mu$g/mL. Each of aqueous solutions was charged in a gel filtration column (product name: Sephacrl S300 HR, manufactured by GE Health Care) as a sample, and eluted using 5 mM Tris-HCl buffer solution (pH: 8.0, 0.15 M NaCl was contained) at a flow rate of 0.5 mL/ min in a temperature condition of 4°C. A non-adsorbed fraction was eluted and detected by an absorption wavelength of 215 nm. In  addition, standard proteins of which molecular weight were different each other were eluted in the same conditions.
In the above-described column chromatography, single peaks were measured at molecular weight of about 160,000 and 130,000 respectively. Each fraction (CM2 non-adsorbed S300 fraction and CM2 adsorbed S300 fraction) was respectively obtained, and subjected to dialysis using water. Then, the fractions were concentrated using a filter for removing particle (product name: Centricut, manufactured by KURABO INDUSTRIES Co., Ltd., molecular weight cut off: 10,000), and 3.9 mg and 2.9 mg of purified samples were respectively obtained. The samples were subjected to Native-PAGE; as a result, single bands were respectively detected. In addition, the samples were subjected to SDS-PAGE; as a result, multiple bands mainly at 46 kDa and 52 kDa were respectively detected. From the above result, it was demonstrated that at least a part of the purified substances formed a complex.

Test Example 1: Measurement of ice crystallization inhibitory activity

An ice crystallization inhibitory activity of each solution obtained in Examples 1 to 6 was measured. Specifically, each solution obtained in Examples 1 to 6 was diluted to adjust a protein concentration as described in Table 1, and then the diluted solutions were mixed with 60 w/v% sucrose aqueous solution in equal amount to obtain 30 w/v% sucrose solution of the sample. In order to evaluate an ice crystallization inhibitory activity, under a microscope having a stage with a function to control a low temperature, 30 w/v% sucrose solution of the sample was cooled down to -40°C and then warmed to -6°C to melt the ice crystals. The ice crystals were observed for 30 minutes with maintaining the temperature at -6°C, and an average area was measured. The average area of ice crystals was measured by analyzing a microscopic image using a commercially available image analysis process soft (Image Factory manufactured by Imsoft) and dividing a total area of the ice crystals in the image by the number of the ice crystals. In addition, with respect to 30 w/v% sucrose aqueous solution as a control, a similar measurement was carried out and an average area of ice crystals was calculated.

An average area of ice crystals is smaller as an ice crystallization inhibitory activity is stronger. Therefore, the average area was divided by the average area of ice crystals of 30 w/v% sucrose aqueous solution as a control, and an ice crystallization inhibitory activity was quantitatively evaluated using the calculated value (RI value) as an indicator. In addition, a concentration of protein in the sample was measured by BCA method, a specific activity was obtained by dividing the reciprocal of RI value by the protein concentration, and a value relative to a crude extract of Example 1 was calculated. The result is demonstrated in Table 1.

[Table 1]

|  | Sample | Protein concentration (mg/mL) | Ice crystallization inhibitory activity (RI value) | Specific activity (Relative value) |
|---|---|---|---|---|
| Example 1 | crude excract | 3.0 | 0.33 | 1 |
| Example 2 | acetone fraction | 0.5 | 0.40 | 5.0 |
| Example 3 | DEAE fraction | 0.4 | 0.29 | 8.5 |
| Example 4 | CM1 fraction | 0.2 | 0.25 | 19.8 |
| Example 5 | CM2 non-adsorbed fraction | 0.12 | 0.33 | 25.0 |
| Example 5 | CM2 adsorbed fraction | 0.14 | 0.37 | 19.1 |
| Example 6 | CM2 non-adsorbed S300 fraction | 0.13 | 0.27 | 28.2 |
| Example 6 | CM2 adsorbed S300 fraction | 0.13 | 0.22 | 34.6 |

From the result described in Table 1, it was clearly demonstrated that an extract which has an excellent ice crystallization inhibitory activity can be obtained from commercially available Dainagon adzuki bean and an ice crystallization inhibitory activity per protein, i.e. specific activity, is remarkably improved by fractionation and purification of Examples 2 to 6.

Test Example 2: Measurement of thermal hysteresis

The CM2 non-adsorbed S300 fraction and CM2 adsorbed S300 fraction obtained in Example 6 were respectively dissolved in water so that a concentration of protein became 0.25 mg/mL, and a thermal hysteresis was measured as follows. Specifically, a glass petri dish was maintained in a temperature of -20°C using a phase contrast microscope (product name: L600A, manufactured by Olympus Corporation) which could control a temperature in a low temperature range, 1 mL of the sample was placed on the glass petri dish, and a temperature was decreased to -40°C at a speed of 100°C/min to form ice crystals. The ice crystals were warmed up to -5°C at a speed of 100°C/min, and then further warmed up at a speed of 5°C/min to be single crystals. Next, the time at which the single crystals started to grow was measured by decreasing a temperature at a speed of 1°C/min, and a thermal hysteresis was calculated in accordance with the following formula.

```
Thermal hysteresis (°C) = [60 - 1] (°C/sec) × measured time
(sec)
```

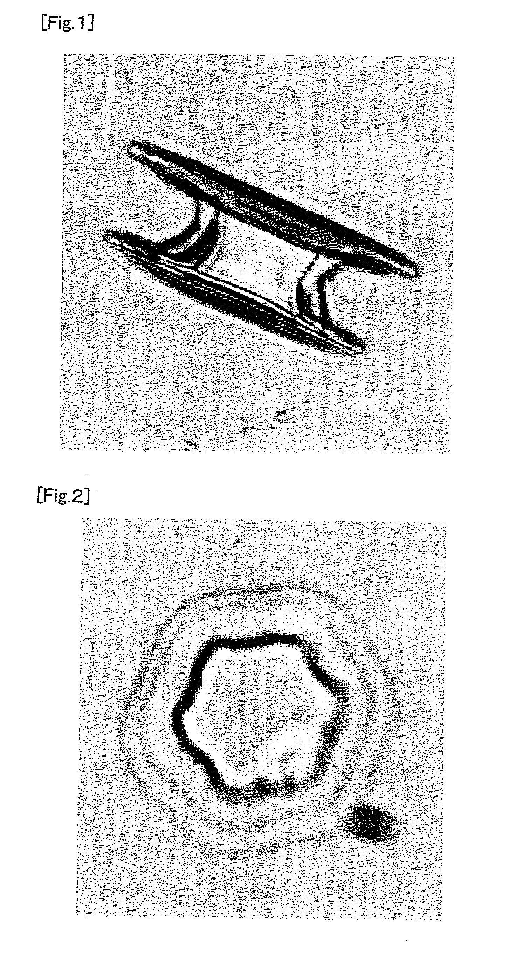

The higher calculated value is, the higher an ice crystallization inhibitory activity is. The result is demonstrated in Table 2. In addition, a magnified photograph of an ice crystal of which figure was controlled by CM2 non-adsorbed S300 fraction is demonstrated as Figure 1, and a magnified photograph of an ice crystal of which figure was controlled by CM2 adsorbed S300 fraction is demonstrated as Figure 2.

[Table 2]

| Sample | Thermal hysteresis (°C) |
|---|---|
| CM2 non-adsorbed S300 fraction | 0.036 |
| CM2 adsorbed S300 fraction | 0.055 |

From the result described in Table 2, it was clearly demonstrated that the purified protein obtained in Example 6 had an excellent thermal hysteresis as an ice crystallization inhibitor.

In addition, as figures 1 and 2, both of ice crystals obtained by adding the purified protein of Example 6 had forms of non-cutting hexagonal, and the forms were different from a flat disc form of an ice crystal obtained from ordinary water or aqueous solution. It was demonstrated by such a change in form that the obtained purified protein bound to a crystal face of an ice crystal. Therefore, the experimental result provides evidence that the purified proteins are ice crystallization inhibitors. The photograph of Figure 1 represents a side direction of a hexagonal crystal.

Test Example 3: Determination of amino-acid sequence of purified protein

The CM2 adsorbed S300 fraction (4.0 $\mu$g) obtained in Example 6 was dissolved in distilled water (5 $\mu$L), and the solution was subjected to a protease treatment in accordance with an ordinary method. Then, the solution was mixed with a sample buffer (product name: EzApply, manufactured by ATTO corporation) (5 $\mu$L) in a volume ratio of 1 : 1, and the mixture was heated at 99°C for 3 minutes. The sample after the above heat treatment was applied on 10 to 20% polyacrylamide gel (product name: e-Page I, manufactured by ATTO corporation), and SDS-PAGE was carried out at 20 mA for 85 minutes. The gel after SDS-PAGE was transferred on a PVDF membrane (product name: Immobilon PSQ, manufactured by Millipore Corporation) by semi-dry method, and CBB staining was carried out. Multiple bands which mainly contained 52 kDa digested by protease were cut out, and amino-acid sequence was determined by Edman method using a protein sequencer (product name: PPSQ-33A, manufactured by Shimadzu Corporation). The determined sequences are demonstrated as SEQ ID NO: 1 to 3.

The sequences had a high homology with a part of an amino-acid sequence of a LEA protein (Late embryogenesis abundant protein) which was an already-known seed protein. It was clearly demonstrated from the above result that the ice crystallization inhibitor obtained from an adzuki bean was a seed protein.

Example 7

Crude extracts were produced from commercially available mung bean, white adzuki bean and adzuki bean produced in Tokachi which belonged to genus Vigna in Leguminosae as similarly to Example 1. A concentration of protein was adjusted to 2.0 mg/mL, and an ice crystallization inhibitory activity was measured similarly to the method of Test Example 1. The result is demonstrated in Table 3.

[Table 3]

| Raw material | Ice crystallization inhibitory activity (RI value) |
|---|---|
| mung bean | 0.24 |
| white adzuki bean | 0.18 |
| adzuki bean produced in Tokachi | 0.33 |

It was demonstrated by the result described in Table 3 that a seed protein obtained from a seed of the above-described plant belonging to genus Vigna in Leguminosae has excellent ice crystallization inhibitory activity.

Example 8

A crude extract obtained from a mung bean in Example 7 was frozen at -20°C, the frozen extract was melted and subjected to centrifugation, and the supernatant was recovered to remove deposition. A concentration of protein in the obtained crude extract was measured by BCA method; as a result, the concentration was 15.5 mg/mL. The pH of the crude extract (200 mL) was adjusted to 11.0 by adding sodium hydroxide. Then, the crude extract was neutralized and the pH was adjusted to 7.0 by adding hydrochloric acid. The mixture was subjected to dialysis using a dialysis membrane (molecular weight cut off: 14,000), and then the solvent was replaced with water to obtain a solution (230 mL). A concentration of protein in the obtained solution was measured by BCA method; as a result, the concentration was 10.0 mg/mL.

Example 9

The crude extract (100 mL) after an alkaline treatment obtained in Example 8 was subjected to ice crystallization inhibitor concentration process (a cold finger (CF) method) described below. Specifically, a cooling bar having a cylinder shape was inserted into the crude extract of Example 8 containing an ice crystallization inhibitor, and the solution around the cooling bar was cooled at a low cooling speed (from 0.5°C to -1.5°C for 16 hours). After 16 hours, ice which generated around the cooling bar and on which ice crystallization inhibitor was adsorbed was recovered. The above process was repeated two times, and a solution (50 mL) after CF treatment was obtained. A concentration of protein in the obtained solution was measured by BCA method; as a result, the concentration was 4.4 µg/mL. The solution was subjected to SDS-PAGE; as a result, a single band was detected at the position of about 48 kDa.

Example 10

To the crude extract (230 mL) after an alkaline treatment obtained in Example 8, acetone which was cooled down to -30°C was added dropwise little by little until an acetone concentration became 25 v/v%. Then, an acetone solution was separated from an insoluble component (insoluble fraction 3). Next, after acetone was added to the acetone solution until an acetone concentration became 50 v/v%, and an acetone solution was separated from an insoluble component (insoluble fraction 4). Further, after acetone was added to the acetone solution until an acetone concentration became 75 v/v%, an acetone solution was separated from an insoluble component (insoluble fraction 5). The above insoluble fractions 3 to 5 were dissolved in 5 mM Tris-HCl buffer solution (pH 8.0). The solutions were subjected to dialysis using a dialysis membrane (molecular weight cut off: 14,000), and then the solvent was replaced with the same buffer solution. With respect to insoluble fraction 3 and insoluble fraction 5, an ice crystallization inhibitory activity could not be confirmed. On the other hand, it was confirmed that insoluble fraction 4 (30 mL) had a strong ice crystallization activity. Therefore, insoluble fraction 4 was recovered as an acetone fraction. A concentration of protein in insoluble fraction 4 was measured by BCA method; as a result, the concentration was 3.0 mg/mL.

Example 11

The acetone fraction (3.0 mg) obtained in Example 10 was charged in a DEAE column (product name: DEAE TOYOPEARL 650M, manufactured by TOSOH Corporation) which was equilibrated using 5 mM Tris-HCl buffer solution (pH 8.0), and eluted at a flow rate of 2.0 mL/min with NaCl gradient of 0 to 0.5 M. A peak was detected using absorption wavelength of 215 nm. An adsorbed fraction which was obtained when a peak was detected was recovered as an active fraction. The obtained fraction was subjected to dialysis and desalination using a dialysis membrane (molecular weight cut off: 14,000), and the solvent was replaced with deionized water. A concentration of protein in the obtained fraction was measured by BCA method; as a result, the concentration was 12 µg/mL (yield: 30 mL).

Test Example 4: Measurement of ice crystallization inhibitory activity

With respect to each solution which was obtained by extracting from a mung bean in Examples 7 to 11, an ice crystallization inhibitory activity was measured by a similar method as Test Example 1. From protein concentrations of each solution measured by BCA method and RI value which is the indicator of an ice crystallization inhibitory activity, a specific activity per protein was calculated in accordance with the formula represented in Test Example 1 and a value relative to the crude extract of Example 7 was calculated. In addition, a total mass of protein extracted from 100 g of a mung bean was calculated from a solution amount and a protein concentration. The result is demonstrated in Table 4 and Table 5.

[Table 4]

| | Sample | Specific activity (Relative value) | Total protein mass (mg) |
|---|---|---|---|
| Example 7 | crude extract from mung bean | 1 | 3100 |

(continued)

|  | Sample | Specific activity (Relative value) | Total protein mass (mg) |
|---|---|---|---|
| Example 8 | crude extract from mung bean after alkaline treatment | 1.3 | 2300 |
| Example 9 | crude extract from mung bean after CF treatment | 1.5 | 1 |

[Table 5]

|  | Sample | Specific activity (Relative value) | Total protein mass (mg) |
|---|---|---|---|
| Example 7 | crude extract from mung bean | 1 | 3100 |
| Example 8 | crude extract from mung bean after alkaline treatment | 1.3 | 2300 |
| Example 10 | acetone fraction | 2 | 89 |
| Example 11 | DEAE fraction | 5.9 | 11 |

As the result demonstrated in Table 4, the ice crystallization inhibitor derived from a mung bean was purified by purification processes of Examples 7 to 9 so that a specific activity became 1.3 times and a protein concentration became 1/3100. In addition, as the result demonstrated in Table 5, the ice crystallization inhibitor derived from a mung bean was purified by purification processes of Examples 10 and 11 so that a specific activity became 5.9 times and a protein concentration became 11/3100.

Further, as the result demonstrated in Tables 4 and 5, an ice crystallization inhibitory activity was increased as much as 1.3 times by an alkaline treatment of Example 8. It was demonstrated by the result that an ice crystallization inhibitory activity can be increased in the condition that protein is denatured, such as an alkaline treatment. The above result is related to the feature of a LEA protein, which is known to prohibit a denatured protein from being aggregated. Accordingly, the following Test Example 5 and Test Example 6 were carried out.

Test Example 5: Inhibiting effect on aggregation of protein using purified fraction

Commercially available LDH: lactate dehydrogenase (15 $\mu$g) was dissolved in 1.0 mL of water. In the solution, 9 $\mu$g of purified mung bean protein after CF treatment obtained in Example 9 was dissolved. An aqueous solution of LDH in which a purified protein after CF treatment was not added was used as control. Each solution was frozen using liquid nitrogen, and the frozen solution was melted at room temperature. An absorbance of the melted solution at 340 nm was measured. The result is demonstrated in Table 6.

[Table 6]

| Sample | Absorbance (340nm) |
|---|---|
| LDH aqueous solution (control) | 0.21 |
| LDH aqueous solution + CF purified protein | 0.06 |

A turbidity of LDH aqueous solution as control was increased, since LDH protein was denatured and aggregated due to freezing and thawing. On the other hand, in case of the solution containing the purified protein after CF treatment derived from a mung bean, an increase of turbidity was remarkably inhibited. It was demonstrated from the result that the purified mung bean protein obtained in Example 9 has an inhibitory effect on aggregation of protein, which inhibitory effect is characteristic of a LEA protein, and the purified mung bean protein can protect a protein and inhibit denaturation of protein due to freezing and thawing.

Test Example 6: Increase of ice crystallization inhibitory activity of crude mung bean extract, associated with inhibitory effect on aggregation

A protein concentration of a crude mung bean extract obtained in Example 7 was adjusted to 60 mg/mL. To 240 $\mu$L of the solution, 30 $\mu$g of the purified mung bean protein after CF treatment obtained in Example 9 was dissolved. The crude mung bean extract to which the purified protein after CF treatment was not added was used as control. Each solution was frozen using liquid nitrogen, and the frozen solution was melted at room temperature. The melted solution was subjected to centrifugation, and then a protein concentration was adjusted to 0.95 mg/mL. An ice crystallization inhibitory activity of the solution was measured by a similar method to Test Example 1. The result is demonstrated in Table 7.

[Table 7]

| Raw material | Ice crystallization inhibitory activity (RI value) |
|---|---|
| crude extract from mung bean (control) | 0.53 |
| crude extract from mung bean + CF purified protein | 0.32 |

An ice crystallization inhibitory activity of the crude mung bean extract was remarkably increased by adding the purified mung bean protein after CF treatment obtained in Example 9 in a slight amount, and freezing and thawing the mixture. Such an increase of an ice crystallization inhibitory activity was not observed when the purified mung bean protein after CF treatment was merely added. Therefore, it was thought that an ice crystallization inhibitory activity was increased by interacting the purified mung bean protein with a protein and the like in the crude extract when stress of freezing and thawing was applied.

Example 12

The DEAE fraction obtained in Example 11 (4.5 mg) was charged in a gel filtration column (product name: Sephacryl S300 HR, manufactured by GE Health Care) as a sample, and eluted using 5 mM Tris-HCl buffer solution (pH: 8.0, 0.15 M NaCl was contained) at a flow rate of 0.5 mL/ min in a temperature condition of 4°C. A non-adsorbed fraction was eluted and detected by an absorption wavelength of 215 nm. In addition, standard proteins of which molecular weight were different each other were eluted in the same conditions. By the above column chromatography, an absorption peak was detected at the same molecular weight (160,000 and 130,000) as the case of Dainagon adzuki bean which was subjected to purification in Example 6.

```
<110>   KANSAI UNIVERSITY

<110>   KANEKA CORPORATION

<120>   ANTIFREEZE SUBSTANCE DERIVED FROM PLANT SEED

<130>   F12-004PCT

<150>   JP 2011-048318
<151>   2011-3-4

<160>   2

<210>   SEQ  ID NO: 1
<211>   25
<212>   PRT
<213>   Dainagon Adzuki Bean

<400>   SEQ ID NO: 1
Met Ala Val Gln Glu Ala Val Ala Gly Lys Lys Glu Ser Val Pro
1               5                   10                  15
Ala Lys Ala His Thr Thr Glu Val Ile His
                20                  25

<210>   SEQ  ID NO: 2
<211>   11
<212>   PRT
<213>   Dainagon Adzuki Bean

<400>   SEQ ID NO: 2
Thr Leu Asn Lys Met Gly Glu Tyr Lys Asp Tyr
1               5                   10

<210>   SEQ  ID NO: 3
<211>   22
<212>   PRT
<213>   Dainagon Adzuki Bean

<400>   SEQ ID NO: 3
Met Lys Glu Gly Lys Asp Ala Thr Leu Asn Lys Met Gly Glu Tyr
1               5                   10                  15
Lys Asp Tyr Thr Ala Glu Lys
```

**Claims**

1.  An ice crystallization inhibitor, comprising a seed protein derived from a plant belonging to genus Vigna in Leguminosae, an allied species thereof or an improved species thereof.

2.  The ice crystallization inhibitor according to claim 1, wherein the plant belonging to genus Vigna in Leguminosae is an adzuki bean or a mung bean.

3.  The ice crystallization inhibitor according to claim 1 or 2, wherein the seed protein is a grain filling protein.

4.  The ice crystallization inhibitor according to any one of claims 1 to 3, wherein the seed protein is a LEA protein: Late embryogenesis abundant protein.

5.  The ice crystallization inhibitor according to claim 4, wherein an amino-acid sequence of the seed protein is a sequence of SEQ ID NO: 1, or an amino-acid sequence corresponding to the sequence of SEQ ID NO: 1 with one or more amino-acid deletions, substitutions or additions and containing an indicative sequence of a protein having an ice crystallization inhibitory activity.

6. The ice crystallization inhibitor according to claim 4, wherein an amino-acid sequence of the seed protein is a sequence of SEQ ID NO: 2, or an amino-acid sequence corresponding to the sequence of SEQ ID NO: 2 with one or more amino-acid deletions, substitutions or additions and containing an indicative sequence of a protein having an ice crystallization inhibitory activity.

7. The ice crystallization inhibitor according to any one of claims 1 to 6, wherein the seed protein forms a complex with other protein.

8. An antibody, specifically responding with the ice crystallization inhibitor according to any one of claims 1 to 7.

9. A composition, comprising the ice crystallization inhibitor according to any one of claims 1 to 7.

10. A food, comprising the ice crystallization inhibitor according to any one of claims 1 to 7.

11. A biological sample protectant, comprising the ice crystallization inhibitor according to any one of claims 1 to 7.

12. A cosmetic, comprising the ice crystallization inhibitor according to any one of claims 1 to 7.

13. A peptide, having a sequence of SEQ ID NO: 2, or an amino-acid sequence corresponding to the sequence of SEQ ID NO: 2 with one or more amino-acid deletions, substitutions or additions and containing an indicative sequence of a protein having an ice crystallization inhibitory activity.

[Fig.1]

[Fig.2]

| | **INTERNATIONAL SEARCH REPORT** | International application No. |
|---|---|---|
| | | PCT/JP2012/055460 |

A.  CLASSIFICATION OF SUBJECT MATTER
*C07K14/415*(2006.01)i, *A23L1/305*(2006.01)i, *A61K8/64*(2006.01)i, *A61K8/97*
(2006.01)i, *A61Q19/00*(2006.01)i, *C07K16/16*(2006.01)i, *C12N1/04*(2006.01)i

According to International Patent Classification (IPC) or to both national classification and IPC

B.  FIELDS SEARCHED

Minimum documentation searched (classification system followed by classification symbols)
C07K14/415, A23L1/305, A61K8/64, A61K8/97, A61Q19/00, C07K16/16, C12N1/04

Documentation searched other than minimum documentation to the extent that such documents are included in the fields searched
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
    CA/BIOSIS/MEDLINE/WPIDS(STN), JSTPlus/JMEDPlus/JST7580(JDreamII),
    UniProt/GeneSeq

C.  DOCUMENTS CONSIDERED TO BE RELEVANT

| Category* | Citation of document, with indication, where appropriate, of the relevant passages | Relevant to claim No. |
|---|---|---|
| Y | JP 2005-104875 A (Kansai University), 21 April 2005 (21.04.2005), claims (Family: none) | 1-13 |
| Y | JP 2007-169246 A (Kansai University), 05 July 2007 (05.07.2007), claims (Family: none) | 1-13 |
| Y | RORAT T., Plant dehydrins - tissue location, structure and function, Cell. Mol. Biol. Lett., 2006, Vol.11, No.4, pp.536-556 | 1-13 |

| ☐ Further documents are listed in the continuation of Box C. | ☐ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 09 May, 2012 (09.05.12) | 22 May, 2012 (22.05.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004083546 A
- JP 2002507889 T
- JP 2004161761 A
- JP 2004024237 A
- JP 2004275008 A
- WO 9222581 A
- WO 9403617 A
- JP 2011048318 A

**Non-patent literature cited in the description**

- *Plant Physiology,* 1999, vol. 119, 1361-1369
- *Biochem. J.,* 1999, vol. 340, 385-391